(19) Europäisches Patentamt
European Patent Office
Office européen des brevets

(11) Veröffentlichungsnummer : **0 105 143**
**B1**

(12) # EUROPÄISCHE PATENTSCHRIFT

(45) Veröffentlichungstag der Patentschrift : **04.03.87**

(51) Int. Cl.⁴ : **C 07 C125/065**

(21) Anmeldenummer : **83107940.5**

(22) Anmeldetag : **11.08.83**

(54) **Verfahren zur Herstellung von N-substituierten Carbamaten.**

(30) Priorität : **08.09.82 DE 3233309**

(43) Veröffentlichungstag der Anmeldung : **11.04.84 Patentblatt 84/15**

(45) Bekanntmachung des Hinweises auf die Patenterteilung : **04.03.87 Patentblatt 87/10**

(84) Benannte Vertragsstaaten : **BE CH DE FR GB LI NL**

(56) Entgegenhaltungen : **DE-B- 1 157 598**

(73) Patentinhaber : **BASF Aktiengesellschaft**
**Carl-Bosch-Strasse 38**
**D-6700 Ludwigshafen (DE)**

(72) Erfinder : **Nestler, Gerhard, Dr.**
**Van-Leyden-Strasse 17**
**D-6700 Ludwigshafen (DE)**
Erfinder : **Merger, Franz, Dr.**
**Max-Slevogt-Strasse 25**
**D-6710 Frankenthal (DE)**

**EP 0 105 143 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein Verfahren zur Herstellung von N-substituierten Carbamaten durch Umsetzung von Carbaminsäureestern mit Alkoholen oder Alkoholderivaten in Gegenwart von Säuren als Katalysatoren.

Aus der DE-AS 1 157 598 ist bekannt, daß die Umsetzung von Carbaminsäureestern mit Olefinen in Gegenwart von wasserfreien Mineralsäuren, organischen Sulfonsäuren oder Lewissäuren N-alkylierte Carbaminsäureester liefert. Bevorzugt werden dabei konzentrierte Schwefelsäure und Bortrifluoridetherat eingesetzt (loc. cit., Spalte 2, Seiten 22-25 und Beispiele 1-12). Nachteilig für dieses Verfahren ist jedoch das Auftreten von Nebenprodukten (Telomere der Olefine) und die dadurch bedingte geringe Ausbeute (10 bis 70 % der Theorie). Außerdem sind die den N-Alkylcarbminsäureestern zugrundeliegenden Olefine mitunter nicht verfügbar bzw. nur äußerst schwierig zugänglich. Zwar wird die Gruppe saurer Ionenaustauscher erwähnt, aber weder Einzelstoffe noch Arbeitsweise und Ausführungsbeispiele beschrieben. Als Lösungsmittel werden Chlorkohlenwasserstoffe, aromatische oder aliphatische Kohlenwasserstoffe erwähnt, in allen Beispielen aber nur Benzol und Toluol und Eisessig verwendet. Es ist ebenso bekannt, daß es unter den stark sauren Alkylierungsbedingungen zu einer teilweisen Telomerisation der eingesetzten Olefine kommt. Diese Telomere können sich wieder mit dem eingesetzten Carbaminsäureester zu N-alkylierten Carbamaten umsetzen (DE-AS 1 157 598, Spalte 3, Zeilen 7-10).

N-substituierte Carbaminsäureester werden zur Zeit hauptsächlich aus den entsprechenden Isocyanaten und Alkoholen oder aus den entsprechenden Aminen und Chlorameisensäureestern bzw. aus N-substituiertem Harnstoff und Alkoholen hergestellt (Houben-Weyl, Methoden der Organischen Chemie, Band 8, Seiten 137-143). Alle diese genannten Verfahren gehen jedoch von den entsprechenden Aminen aus, die bekanntlich meistens nur schwierig und aufwendig hergestellt werden können, und sie benötigen teilweise das toxische Phosgen.

Es wurde nun gefunden, daß man N-substituierte Carbamate der Formel I,

$$R^2-N(H)-C(=O)-O-R^1 \qquad (I)$$

worin die Reste $R^1$ und $R^2$ gleich oder verschieden sein können und jeweils einen Alkylrest mit 1 bis 12, insbesondere 1 bis 8 Kohlenstoffatomen, der als Glieder der Kohlenstoffkette noch 1 oder 2 Carboxylgruppen enthalten kann, bezeichnen und $R^2$ auch einen gegebenenfalls mit Alkylgruppen, insbesondere 1 oder 2 Alkylgruppen mit jeweils 1 bis 4 Kohlenstoffatomen substituierten mono- oder bicyclischen gesättigten Cycloalkylrest mit 5 bis 8 Kohlenstoffatomen, einen Cycloalkenylrest mit 5 bis 8 Kohlenstoffatomen oder einen Aralkylrest mit 7 bis 14 Kohlenstoffatomen bezeichnet, aus Carbaminsäureestern in Gegenwart von Säuren als Katalysatoren, vorteilhaft erhält, wenn man Carbaminsäureester der Formel II

$$H-N(H)-C(=O)-OR^1 \qquad (II)$$

mit Alkoholen oder Alkoholderivaten der Formel III

$$R^2-O-R^3 \qquad (III)$$

worin $R^3$ die für $R^1$ angegebene Bedeutung besitzt und darüber hinaus für ein Wasserstoffatom oder den Rest

$$-C(=O)-R^4$$

steht, worin $R^4$ die für $R^1$ angegebene Bedeutung besitzt, umsetzt.

Die Umsetzung kann für den Fall der Verwendung des Carbaminsäureethylesters und des tert.-Butylacetats durch die folgenden Formeln wiedergegeben werden :

$$NH_2CO_2C_2H_5 + (CH_3)_3CO-C(=O)-CH_3 \longrightarrow H_3C-C(CH_3)_2-NHCO_2C_2H_5 + CH_3CO_2H$$

Im Vergleich zu den bekannten Verfahren liefert das Verfahren nach der Erfindung überraschend auf einfacherem und wirtschaftlicherem Wege N-substituierte Carbaminsäureester in besserer Ausbeute, Raum-Zeit-Ausbeute und Reinheit. Im Gegensatz zu den Umsetzungen entsprechend der DE-AS 1 157 598 (siehe Vergleichsbeispiel 1) wird bei der erfindungsgemäßen Alkylierung die Bildung von Olefinoligomeren und der entsprechenden N-substituierten Carbaminsäureester weitgehend verhindert.

Es ist außerdem überraschend, daß es dabei nicht zur Desaktivierung (Neutralisation) des Katalysators durch freiwerdende Amine kommt. Entsprechend dem Stand der Technik (« The Chemistry of Open-Chain Nitrogen Compounds » Vol. 1, Seite 261 (Gleichung 107), W.A. Benjamin Inc., 1965, New York), hätte man nämlich anstelle der dort beschriebenen sauren Hydrolyse eine säurekatalysierte Alkoholyse der Carbamate entsprechend der folgenden Gleichungen erwartet :

$$NH_2\overset{O}{\overset{\|}{C}}OR^1 + R^2OH \longrightarrow NH_3 + R^2O\overset{O}{\overset{\|}{C}}OR^1$$

Wie die Beispiele 2 und 3 zeigen, konnte jedoch weder eine Desaktivierung des Katalysators festgestellt werden, noch die Bildung von Carbonaten nachgewiesen werden.

Bei der Umsetzung der Carbaminsäureester mit Estern hätte man außerdem die Bildung von acylierten Carbaminsäureestern erwarten müssen :

$$H_2N-\overset{O}{\overset{\|}{C}}-OR^1 + R^2O-\overset{O}{\overset{\|}{C}}-R^4 \longrightarrow R^4-\overset{O}{\overset{\|}{C}}-NH-\overset{O}{\overset{\|}{C}}-OR^1 + R^2OH$$

Es ist nämlich bekannt, daß NH-Verbindungen mit Carbonsäureestern zu den entsprechenden N-acylierten Verbindungen umgesetzt werden können (Organikum (1971), Seiten 449-455, insbesondere 451 und 455).

Es ist weiterhin überraschend, daß keine wesentliche Bildung von Carbonaten beobachtet wird ; denn es ist aus DE-OS 3 024 554 bekannt, daß man Carbamidsäureester mit Alkoholen bei einer Temperatur oberhalb 140 °C zu Carbonaten umsetzt, wobei man während der Umsetzung den gebildeten Ammoniak abtrennt.

In der DE-AS 1 157 598 (Spalte 2, Zeile 19) wird zwar die Verwendung von sauren Ionenaustauschern vorgeschlagen, doch da keine Angaben über den Austauscher gemacht werden, mußte angenommen werden, daß es sich dabei um die zu dieser Zeit üblichen Austauscherharze vom Gel-Typ handelt. Wie jedoch Vergleichsbeispiel 1 zeigt, lassen sich mit diesen Austauscherharzen vom Gel-Typ nur unbefriedigende Ergebnisse erzielen.

Als Ausgangsstoffe III werden Alkohole, Äther oder Ester verwendet. Der Ausgangsstoff III kann mit dem Carbaminsäureester II in stöchiometrischer Menge, im Überschuß oder Unterschuß, vorzugsweise in einem Verhältnis von 0,1 bis 5, insbesondere bei Verwendung von Alkoholen 0,3 bis 1,5, bei Verwendung von Äthern 0,3 bis 1,5, bei Verwendung von Estern 0,3 bis 2 Mol Ausgangsstoff III je Mol Carbaminsäureester II umgesetzt werden.

Beispielsweise sind folgende Carbaminsäureester II geeignet :

Carbaminsäuremethylester ; homologe Ethyl-, Propyl-, Butyl-, Nonyl-, Decyl-, Isopropyl-, Isobutyl-, Octyl-, Cyclohexyl, Pentyl-, Heptyl-, Cyclopentyl-, n-Hexyl-ester ; Vorzugsweise verwendet man den Carbaminsäuremethyl-, -ethyl-, -propyl-, -n-butyl-, -n-octyl- oder -2-ethylhexyl-ester.

Es können z. B. folgende Alkohole als Ausgangsstoffe III verwendet werden :

tert.-Amyl-, tert.-Butyl-, Cyclopentyl-, 1-Methylcyclopentyl-1-, Cyclohexyl-, Norbornyl-, Benzyl-, 2-Ethylhexyl-2-, α-Methylbenzyl-, 1-Methylcyclohexyl-1-alkohol ; vorzugsweise tert. Butanol, 1,1-Dimethylpropanol-1, 3-Methyl-3-pentanol, 3-Methylhexanol-3, Diacetonalkohol oder Benzhydrol.

Als Ausgangsstoffe III kommen beispielsweise die folgenden Äther in Betracht : Äther, die durch die Verätherung eines der vorgenannten, als Beispiele aufgeführten Alkohole mit sich selbst gebildet werden ; Äther, die durch die Verätherung von 2 der vorgenannten, als Beispiele aufgeführten Alkohole gebildet werden, z. B. die bevorzugten Methyl-tert.-butylether, Isobutyl-tert.-butylether oder Propyl-tert.-amylether.

Als Ausgangsstoffe III sind z. B. folgende Ester geeignet : Ester der Essigsäure mit einem der vorgenannten, als Beispiele aufgeführten Alkohole ; homologe Ester der Ameisensäure, Propion-, Butter-, Isobutter-, Valerian-, Isovalerian-, Capronsäure ; bevorzugt sind tert. Butylacetat, α-Methyl-benzylacetat, Norbornylacetat, tert. Butylformiat.

Die Umsetzung wird im allgemeinen bei einer Temperatur von 26 bis 160 °C, vorzugsweise zwischen 40 und 140 °C, insbesondere 50 bis 120 °C, mit Unterdruck, Überdruck oder zweckmäßig drucklos, kontinuierlich oder diskontinuierlich durchgeführt. Die Verweilzeit beträgt vorzugsweise 1 bis 20, insbesondere von 4 bis 16 Stunden. Man kann die Umsetzung ohne zusätzliche Lösungsmittel oder in Gegenwart zusätzlicher unter den Reaktionsbedingungen inerter, organischer Lösungsmittel durchführen. Als Lösungsmittel kommen z. B. in Frage : aromatische Kohlenwasserstoffe, z. B. Toluol, Äthylbenzol, o-, m-, p-Xylol, Isopropylbenzol, Nitrobenzol, Chlorbenzol ; cyclische Äther, z. B. Tetrahydrofuran, Dioxan ; aliphatische oder cycloaliphatische Kohlenwasserstoffe, z. B. Pentan, Heptan, Benzinfraktionen innerhalb eines Siedepunktintervalls von 70 bis 190 °C, Cyclohexan, Methylcyclohexan, Dekalin, Petroläther, Hexan, Ligroin, Nitromethan und entsprechende Gemische. Bevorzugt sind Essigsäure, Benzol, Toluol, Chlorbenzol, Cyclohexan oder Dioxan. Wird die Reaktion mit Alkoholen III durchgeführt und das entstehende Wasser mittels azeotroper Destillation entfernt, so verwendet man als Schleppmittel vorzugsweise Benzol, Toluol, Chlorbenzol oder Cyclohexan. Die Menge des eingesetzten Lösungs- bzw. Schleppmittels beträgt zweckmäßig von 50 bis 10 000 Gewichtsprozent, vorzugsweise von 100 bis 1 000 Gewichtsprozent, bezogen auf Ausgangsstoff II.

Die Umsetzung wird in Gegenwart von Säure als Katalysator vorteilhaft mit einer Menge von 0,01 bis 10, insbesondere von 0,1 bis 2 Äquivalenten Säure, bezogen auf 1 Mol Ausgangsstoff II, durchgeführt. Es können Lewis-Säuren oder zweckmäßig organische oder insbesondere anorganische Säuren verwendet werden. Anstelle einbasischer Säuren können auch äquivalente Mengen mehrbasischer Säuren zur Anwendung gelangen. Beispielsweise sind folgende Säuren geeignet : Chlorwasserstoff, Bromwasserstoff, Jodwasserstoff, Schwefelsäure, Phosphorsäure, Sulfonsäuren wie Methansulfonsäure, Trifluormethansulfonsäure, Benzol- und p-Toluolsulfonsäure ; Bor enthaltende Säuren wie Borfluorwasserstoffsäure ; saure Ionenaustauscher oder entsprechende Gemische. Bevorzugt sind Schwefelsäure, p-Toluolsulfonsäure, Methansulfonsäure und sulfonsaure organische Ionenaustauscher.

Als saure Katalysatoren werden bevorzugt organische, saure Kationenaustauscher, vorteilhaft Harze aus sulfoniertem Polystyroldivinylbenzol oder sulfonierten, vernetzten Styrolpolymeren ; perfluorierte, organische Sulfonsäuren ; sulfonsaure Phenolformaldehyd- oder Benzolformaldehydharze oder Copolymerisate aus Tetrafluorethylen und Vinylsulfonylfluorid verwendet. Vorzugsweise kommen sulfonierte Polystyroldivinylbenzolaustauscher in Frage. Die Austauscher liegen in der Säureform und nicht als Salz vor. Der Katalysator kann sowohl makroporöse als auch gelartige Struktur besitzen. Er hat vorteilhaft eine Korngröße von 5 bis 2 000, vorzugsweise von 10 bis 1 500, im Falle von gelartigen Katalysatoren insbesondere von 10 bis 200 Mikrometer, und eine Gesamtoberfläche von 10 bis 300 $m^2/g$, im Falle der makroporösen Katalysatoren insbesondere eine innere Oberfläche von 10 bis 200 $m^2/g$. Geeignet sind z. B. Austauscherharze, wie sie unter der Bezeichnung (R)LEWASORB AC-10, (R)Amberlyst-15, (R)LEWATIT SPC-118, (R)LEWATIT SPC-108 oder (R)Nafion im Handel erhältlich sind. Man entwässert sie zweckmäßig vor der Verwendung in üblicher Weise, z. B. durch Erhitzen auf 100 °C bis 110 °C im Vakuum. Die Entwässerung kann jedoch auch durch Verdrängen mit hydrophilen organischen Flüssigkeiten und anschließendem Erhitzen auf 100 °C bei vermindertem Druck oder durch azeotrope Destillation mit einer organischen Flüssigkeit erfolgen.

Der Katalysator in Gestalt eines Ionenaustauschers kann in beliebiger diskontinuierlicher oder kontinuierlicher Arbeitsweise, z. B. in Gestalt eines Festbettes, verwendet werden. Er befindet sich vorteilhaft während der Umsetzung in Suspension, in der Regel im sich bildenden Reaktionsgemisch. Zweckmäßig legt man einen Anteil an Carbaminsäureester II und Katalysator vor und suspendiert den Katalysator in dem Gemisch unter guter Durchmischung. Bevorzugt ist eine Menge von 5 bis 200, insbesondere von 10 bis 100 Gew.-% Ionenaustauscher, bezogen auf die Gewichtsmenge Carbaminsäureester II.

Die Reaktion kann wie folgt durchgeführt werden : Ein Gemisch von Ausgangsstoff II und III, Katalysator und gegebenfalls Lösungsmittel wird während der Reaktionszeit bei der Reaktionstemperatur gehalten. Man kann auch zu einem Gemisch aus Carbaminsäureester II, Säure und gegebenenfalls einem Lösungsmittel unter Rühren bei der Reaktionstemperatur den Ausgangsstoff III kontinuierlich zugeben. Nach Abtrennung des Katalysators kann man den Endstoff in üblicher Weise, z. B. durch Filtration und Destillation, isolieren. Bei der Umsetzung mit Alkoholen III kann das bei der Reaktion entstehende Wasser gegebenenfalls durch azeotrope Destillation mit z. B. Benzol entfernt werden.

Die Reaktion kann im Falle der Verwendung von Ionenaustauschern als Katalysatoren und im kontinuierlichen Betrieb im Festbett oder vorteilhaft wie folgt durchgeführt werden : Ein flüssiges Gemisch von Carbaminsäureester II, Ausgangsstoff III und gegebenenfalls Lösungsmittel, wird bei der Reaktionstemperatur und dem Reaktionsdruck durch eine Suspension des Katalysators im Ausgangsgemisch bzw. Reaktionsgemisch geleitet und filtriert. Dann wird aus dem Reaktionsgemisch der Endstoff in üblicher Weise, z. B. durch Destillation, abgetrennt. Die Filtrierung erfolgt zweckmäßig vor Austritt der Suspension aus dem Reaktor.

Eine weitere Ausführungsform der Umsetzung mit Alkohol III besteht darin, daß man zu einem Gemisch aus Carbaminsäureester II und Säure bei der Reaktionstemperatur ein Gemisch aus Alkohol III und einem Säureanhydrid kontinuierlich zugibt und anschließend das Gemisch bei der entsprechenden Reaktionstemperatur rührt. Als Säureanhydride werden in der Regel Anhydride von Carbonsäuren, vorteilhaft Alkancarbonsäure, insbesondere von 1-6 Kohlenstoffatomen, z. B. der Essigsäure, verwendet ; Mengen von 0,5 bis 1,1, insbesondere 0,8 bis 1 Mol Anhydrid je Mol Ausgangsstoff III, kommen in Frage. Obwohl bekannt ist, daß die Umsetzung von Carbaminsäureestern mit Anhydriden zu acylierten Carbamaten führt (Beilstein Organische Chemie, Band 3 (4. Ausg. 1921), Seiten 26 und 27), konnte die Bildung der entsprechenden acetylierten Carbaminsäureestern nur in geringer Menge, im allgemeinen nur bis zu 2 Gew.-%, bezogen auf Ausgangsstoff II, beobachtet werden.

Die nach dem Verfahren der Erfindung herstellbaren N-substituierten Carbamate I sind Wirkstoffe und wertvolle Ausgangsstoffe für die Herstellung von Farbstoffen, Schädlingsbekämpfungsmitteln und

Pharmazeutika. Durch Hydrolyse der Carbaminsäureester können die entsprechenden Amine hergestellt werden, die ebenfalls wichtige Ausgangsprodukte bei der Synthese von Wirkstoffen darstellen. Bezüglich der Verwendung wird auf die vorgenannten Veröffentlichungen und Ullmanns Encyklopädie der technischen Chemie, Band 5, Seiten 73 bis 76, verwiesen.

Vergleichsbeispiel 1

In einem Rührautoklaven wurde ein Gemisch aus 89 g Carbaminsäureethylester, 20 g Schwefelsäure und 70 g Isobuten unter Rühren auf 70 °C erhitzt und 5 Stunden bei dieser Temperatur gerührt. Nach Beendigung der Umsetzung wurde der Katalysator abgetrennt und das Reaktionsgemisch gaschromatographisch analysiert. Es enthielt neben dem gewünschten Produkt noch 5 % Diisobuten und Triisobutene. Durch Destillation erhielt man 127 g (70 % der Theorie, bezogen auf Isobuten) N-tert.-Butylcarbaminsäureethylester vom Kp. 71-72 °C/20 mbar.

Beispiel 1

In einem Rührautoklaven wurde ein Gemisch aus 89 g Carbaminsäureethylester, 20 g Lewasorb AC-10 und 116 g tert.-Butylcetat auf 70 °C erhitzt und 5 Stunden bei dieser Temperatur gerührt. Nach Beendigung der Umsetzung wurde der Katalysator abgetrennt und das Reaktionsgemisch gaschromatographisch analysiert. Es enthielt neben dem gewünschten Produkt 0,6 % Diisobuten. Durch Destillation erhielt man 134 g (92 % der Theorie) N-tert.-Butylcarbaminsäureethylester vom Kp. 71-72 °C/20 mbar.

Beispiel 2

In einem Rührreaktor wurde ein Gemisch aus 89 g Carbaminsäureethylester und 40 g Lewatit SPC-118 auf 80 °C erhitzt und anschließend wurde innerhalb von 40 Minuten ein Gemisch aus 34 g 3-Methyl-3-pentanol und 34 g Acetanhydrid zudosiert. Nach 5 Stunden Rühren bei 80 °C wurde der Katalysator abgetrennt und destilliert. Man erhielt 49 g (85 % der Theorie) N-(1-Ethyl-1-methylpropyl) carbaminsäureethylester vom Kp. 104-106 °C/20 mbar.

Beispiel 3

In einem mit einem Wasserabscheider ausgerüsteten Rührreaktor wurde ein Gemisch aus 89 g Carbaminsäureethylester, 22 g Lewatit SPC-118, 92 g Benzhydrol und 100 ml Benzol auf 80 °C erhitzt (Rückfluß) und 7 Stunden bei dieser Temperatur gehalten. Nach Beendigung der Umsetzung wurde der Katalysator abgesaugt; dann wurden Benzol und nicht umgesetzter Carbaminsäureethylester destillativ abgetrennt. Der Rückstand wurde aus Ethanol umkristallisiert. Man erhielt 109 g (86 % der Theorie) N-(α-Phenyl-benzyl-) carbaminsäureester mit Fp. 125 °C.

Beispiel 4

In einem Rührautoklaven wurde ein Gemisch aus 117 g Carbaminsäure-n-butylester, 20 g Schwefelsäure (96 Gew.%) und 88 g Methyl-tert.-butylether auf 60 °C erhitzt und 15 Stunden bei dieser Temperatur gerührt. Nach Beendigung der Umsetzung wurde das Gemisch mit Wasser gewaschen und destilliert. Man erhielt 135 g (78 % der Theorie) N-tert.-Butylcarbaminsäure-n-butylester vom Kp. 110-111 °C/20 mbar.

**Patentanspruch**

Verfahren zur Herstellung von N-substituierten Carbamaten der Formel I

$$R^2-\overset{H}{\overset{|}{N}}-\overset{O}{\overset{\|}{C}}-O-R^1 \qquad (I)$$

worin die Reste $R^1$ und $R^2$ gleich oder verschieden sein können und jeweils einen Alkylrest mit 1 bis 12 Kohlenstoffatomen, der als Glieder der Kohlenstoffkette noch 1 oder 2 Carboxylgruppen enthalten kann, bezeichnen und $R^2$ auch einen gegebenenfalls mit Alkylgruppen substituierten mono- oder bicyclischen gesättigten Cycloalkylrest mit 5 bis 8 Kohlenstoffatomen, einen Cycloalkenylrest mit 5 bis 8 Kohlenstoffatomen oder einen Aralkylrest mit 7 bis 14 Kohlenstoffatomen bezeichnet, aus Carbaminsäureestern in Gegenwart von Säuren als Katalysatoren, dadurch gekennzeichnet, daß man Carbaminsäureester der Formel II

$$H-\overset{H}{\overset{|}{N}}-\overset{O}{\overset{\|}{C}}-OR^1 \qquad (II)$$

mit Alkoholen oder Alkoholderivaten der Formel III

$$R^2—O—R^3 \qquad (III)$$

worin $R^3$ die für $R^1$ angegebene Bedeutung besitzt und darüber hinaus für ein Wasserstoffatom oder den Rest

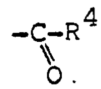

steht, worin $R^4$ die für $R^1$ angegebene Bedeutung besitzt, umsetzt.

**Claim**

A process for the preparation of an N-substituted carbamate of the formula I

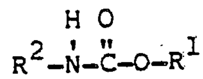
(I)

where the radicals $R^1$ and $R^2$ may be identical or different and are each alkyl of 1 to 12 carbon atoms which may additionally contain 1 or 2 carboxyl groups as members of the carbon chain, and $R^2$ may also denote an optionally alkylsubstituted monocyclic or bicyclic saturated cycloalkyl of 5 to 8 carbon atoms, cycloalkenyl of 5 to 8 carbon atoms or aralkyl of 7 to 14 carbon atoms, from a carbamate in the presence of an acid as catalyst, wherein a carbamate of the formula II

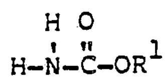
(II)

is reacted with an alcohol or alcohol derivative of the formula III

$$R^2—O—R^3 \qquad (III)$$

where $R^3$ has the same meanings as $R^1$, and additionally denotes hydrogen or

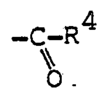

where $R^4$ has the same meanings as $R^1$.

**Revendication**

Procédé de préparation de carbamates N-substitués de la formule I

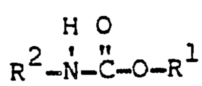
(I)

dans laquelle les substituants $R^1$ et $R^2$, qui peuvent être identiques ou différents, désignent chacun un radical alkyle en $C_1$ à $C_{12}$, qui peut comprendre un ou deux groupes carboxyle comme maillons de la chaîne carbonée, $R^2$ pouvant de plus désigner un groupe cycloalkyle saturé mono- ou bicyclique en $C_5$ à $C_8$, éventuellement substitué par des radicaux alkyle, un groupe cycloalcényle en $C_5$ à $C_8$ ou un groupe aralkyle en $C_7$ à $C_{14}$, à partir d'esters de l'acide carbamique en présence d'un acide comme catalyseur, caractérisé en ce que l'on fait réagir des esters de l'acide carbamique de la formule II

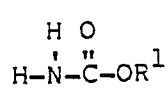
(II)

avec des alcools ou des dérivés d'alcools de la formule III

$$R^2—O—R^3 \qquad (III),$$

dans laquelle $R^3$ possède la signification définie pour $R^1$ ou désigne un atome d'hydrogène ou un groupe

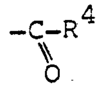

où $R^4$ possède la signification définie pour $R^1$.